# EUROPEAN PATENT APPLICATION

(11) **EP 4 616 799 A1**
(43) Date of publication of application: **17.09.2025**
(21) Application number: 23912613.9
(22) Date of filing: 29.11.2023
(51) Int. Cl.: A61B 5/145, A61B 5/155, A61B 5/157

(54) **SENSOR APPLICATOR ASSEMBLY FOR BLOOD GLUCOSE METER**

(30) Priority: 30.12.2022 KR 20220190422
(71) Applicant: i-Sens, Inc., Seoul 06646 (KR)
(72) Inventor: CHOI, Won Seok, Seoul 06646 (KR); CHOI, Hyun Ho, Seoul 06646 (KR); CHO, Hee Gyung, Seoul 06646 (KR)
(74) Representative: BCKIP Part mbB
(86) International application number: PCT/KR2023/019475
(87) International publication number: WO 2024/143938

(57) **Abstract**

Provided is a sensor applicator assembly for a blood glucose monitoring, comprising an applicator, and a body attachment unit disposed inside the applicator and comprising a housing including an upper frame and a lower frame. The lower frame comprises a recess portion formed throughout an entire section along a perimeter direction.

## Description

### Technical Field

The present disclosure relates to a sensor applicator assembly for a blood glucose monitoring. More specifically, the present disclosure relates to a sensor applicator assembly for a blood glucose monitoring that may enable stable coupling and decoupling operations between an applicator and a body-attachable unit and easy manufacturing and component management of the body-attachable unit.

### Background Art

Diabetes is a chronic disease that considerably occurs to modern people. Diabetes occurs when an absolutely large amount of a sugar component is in blood because a balance between the blood and sugar is not redressed as insulin formed in pancreas is absolutely or relatively insufficient due to various causes such as obesity, stress, a poor diet, and innate heredity.

Generally, the blood contains a specific concentration of glucose, and a tissue cell gains energy therefrom.

However, when glucose increases beyond what the body needs, glucose cannot be properly stored in the liver, intestines, muscles, or fat cells, but is accumulated in the blood, leading a diabetic patient to maintain a significantly higher blood glucose level than a non-diabetic person. As excess blood glucose passes through tissues and is excreted in the urine, the sugar required for each body tissue becomes insufficient, causing abnormalities in each body tissue.

Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear.

In order to diagnose diabetes and manage diabetes to prevent developing the complications, systematic measurement and treatment of the blood glucose may be done simultaneously.

For the diabetic patients and people having sugar measured to be more than normal in blood, multiple medical device manufacturers provide various types of blood glucose monitoring for measuring the blood glucose.

A scheme of a blood glucose monitoring includes a scheme of gathering blood from a fingertip of a user to perform blood glucose measurement on a one-time basis and a scheme of being attached to a stomach and an arm of the user to continuously perform the blood glucose measurement.

In a case of the diabetic patients, a hyperglycemia state and a hypoglycemia state generally alternate with each other. An emergency may occur in the hypoglycemia state, and the patient may lose consciousness or may die when the hypoglycemia state lasts long without supply of sugar. Thus, immediate detection of the hypoglycemia state is greatly important for the diabetic patients. However, accurate identification thereof is limited with a blood gathering-type blood glucose monitoring that intermittently measures the blood glucose.

Recently, in order to overcome such limitation, a continuous glucose monitoring (CGM) system that is inserted in a human body to measure a value of the blood glucose at intervals of several minutes is being developed. In order to minimize a pain or an aversion of the user, which is caused by blood gathering, the CGM system may insert a needle-shaped sensor into a region such as a stomach and an arm at which the pain is relatively less and then continuously measure the blood glucose.

The CGM system includes a body-attachable unit that is attached to a body of the user to measure blood glucose information and transmits the measured blood glucose information and a terminal that provides the blood glucose information received from the body-attachable unit to the user, allowing the user to monitor and manage the blood glucose information.

Here, the body-attachable unit includes a sensor module inserted in and attached to skin of the body of the user and measuring blood glucose from blood fluid of the user and a transmitter transmitting a level of the blood glucose measured by the sensor module to the terminal, and the sensor module is provided with a sensor probe formed in a needle shape to be inserted into subcutaneous fat and to extract the interstitial fluid (ISF).

An applicator is used to insert and attach the body-attachable unit to the body.

The applicator, as a configuration to insert and attach the body-attachable unit to the body, inserts and attaches the body-attachable unit to the body by the user operation, and by detaching and removing the applicator from the body in a state in which the body-attachable unit is inserted and attached to the body, the body-attachable unit is only allowed to remain attached to the body.

To implement the operations, the body-attachable unit is configured to be coupled to and decoupled from the applicator, and for the mutual coupling and decoupling between the body-attachable unit and the applicator, various elements, which are manufactured in different forms by each manufacturer, are used. A general body-attachable unit and an applicator according to a general technology are difficult to be manufactured due to complicated configurations thereof, and there is a problem in which the shapes and positions of various elements should be changed even for minor design modifications. Particularly, coupling or decoupling between the body-attachable unit and the applicator are not stably performed, causing pain during attachment of the body-attachable unit to the body or resulting in an unstable attachment of the body-attachable unit to the body.

### Detailed Description of the Invention

### Technical Goals

The present disclosure is to solve a problem of a general technology and provide a sensor applicator assembly for a blood glucose monitoring that may enable stable coupling and decoupling operations between the applicator and a body-attachable unit, allow easy manufacturing and component management of the body-attachable unit, enhance design flexibility, and enable easier product diversification.

### Technical solutions

Provided is a sensor applicator assembly for a blood glucose monitoring comprising an applicator and a body-attachable unit disposed inside the applicator and comprising a housing including an upper frame and a lower frame. The lower frame may comprise a recess portion formed throughout an entire section along a perimeter direction.

Here, a plunger disposed inside a main case of the applicator may be further comprised. The plunger may be coupled to a fixing hook that is coupled to the body-attachable unit at a first position inside the main case and decoupled from the body-attachable unit at a second position positioned in a downward direction from the first position.

In addition, the body-attachable unit may comprise a sensor member, first end portion of which is disposed inside the housing including the upper frame and the lower frame, and another end portion of which protrudes outside the housing.

In addition, first end portion of the fixing hook may be coupled to the plunger and another end portion may be engaged with the recess portion. The fixing hook may comprise at least one of the first end portion coupled to the plunger.

In addition, the lower frame may comprisea first area coupled to a side wall of the upper frame to be connected in a planar manner without a step and a second area formed with a step in an inward direction from the first area.

In addition, the lower frame may comprise a first area coupled to a side wall of the upper frame to be connected in a planar manner without a step and a second area formed in a shape having an inclined surface as a corner portion extended to a downward direction from the first area is chamfered.

In addition, the plunger may comprise a sensor accommodating portion in which the body-attachable unit is inserted and accommodated. The fixing hook may be coupled to an edge of the sensor accommodating portion and formed to allow first end portion to be pivotable around a hinge axis to be engaged with or disengaged from the recess portion.

In addition, the plunger may move from the first position to the second position within the main case. A hook guide portion may be formed on an inner surface of the main case to press the fixing hook in a direction in which the recess portion is engaged or to release pressure depending on a position of the plunger.

In addition, the plunger may be coupled to an elastic member that applies an elastic force to the fixing hook in a direction in which the fixing hook is disengaged from the recess portion.

Meanwhile, the present disclosure provides a sensor applicator assembly for a blood glucose monitoring that may comprise an applicator and a body-attachable unit disposed inside the applicator and comprising a housing including an upper frame and a lower frame. The lower frame may comprise a recess portion formed in a shape extending from one side of an outer surface of the lower frame through a bottom surface to another side of the outer surface.

Here, the body-attachable unit may comprise a sensor member, first end portion of which is disposed inside the housing including the upper frame and the lower frame, and another end portion of which protrudes outside the housing.

In addition, the recess portion may be formed in a concave groove shape concavely recessed on the bottom surface of the lower frame to cross a bottom surface of the housing in a direction towards both side ends.

In addition, a plunger disposed inside a main case of the applicator may be further comprised. The plunger may further comprise a fixing hook coupled to be engaged with or disengaged from the recess portion and an elastic member that applies an elastic force to the fixing hook in a direction in which the fixing hook is disengaged from the recess portion.

### Effects of the Invention

According to the present disclosure, by forming a recess portion of a body-attachable unit, which is coupled to an applicator, in a continuous shape, it is possible to easily manufacture the body-attachable unit and manage components of the body-attachable unit, enhance design flexibility with regard to the applicator, and easily manufacture and manage products with various specifications and structures.

Furthermore, it is possible to further stabilize operational performance of the applicator and body attachment performance of the body-attachable unit by providing a more stable coupling and decoupling structure between the body-attachable unit and the applicator.

### Brief Description of Drawings

FIG. 1 is illustrating an example of usage of a blood glucose monitoring.
FIG. 2 is an exploded perspective view of an applicator for a blood glucose monitoring according to an example embodiment of the present disclosure.
FIG. 3 is a cross-sectional view illustrating a body-attachable unit installed in an applicator for a blood glucose monitoring according to an example embodiment of the present disclosure.
FIG. 4 is for illustrating an action of a shooting plate provided in an applicator for a blood glucose monitoring according to an example embodiment of the present disclosure.
FIGS. 5 and 6 are perspective views illustrating an assembly of a plunger of an applicator for a blood glucose monitoring and a needle separation unit according to an example embodiment of the present disclosure.
FIG. 7 is a cross-sectional view illustrating an assembly of a plunger of an applicator for a blood glucose monitoring and a needle separation unit according to an example embodiment of the present disclosure.
FIGS. 8 through 13 are illustrating a process of attaching a body-attachable unit to a body of a user by using an applicator for a blood glucose monitoring according to an example embodiment of the present disclosure.
FIG. 14 is a perspective view illustrating a coupling structure of a plunger and a body-attachable unit according to an example embodiment of the present disclosure.
FIGS. 15 through 18 are exemplary diagrams illustrating various forms of a recess portion formed on a body-attachable unit according to an example embodiment of the present disclosure.
FIG. 19 is a schematic perspective view illustrating a form of a hook guide portion formed on an inner surface of a main case according to an example embodiment of the present disclosure.
FIG. 20 is a cross-sectional view illustrating a coupling structure of a plunger and a body-attachable unit at a first position within an applicator according to an example embodiment of the present disclosure.
FIG. 21 is a cross-sectional view illustrating a coupling structure of a plunger and a body-attachable unit at a second position within an applicator according to an example embodiment of the present disclosure.

### Mode for Carrying Out the Invention

Hereinafter, a desired example embodiment of the present disclosure will be described in detail with reference to the accompanying drawings. To begin with, it should be noted that, for adding a reference numeral to an element in each drawing, the same elements have the same reference numeral if possible although illustrated in different drawings. Also, for description of the present disclosure, a detailed description of an associated element or function known to the public will be omitted when determined as obscuring the gist of the present disclosure.

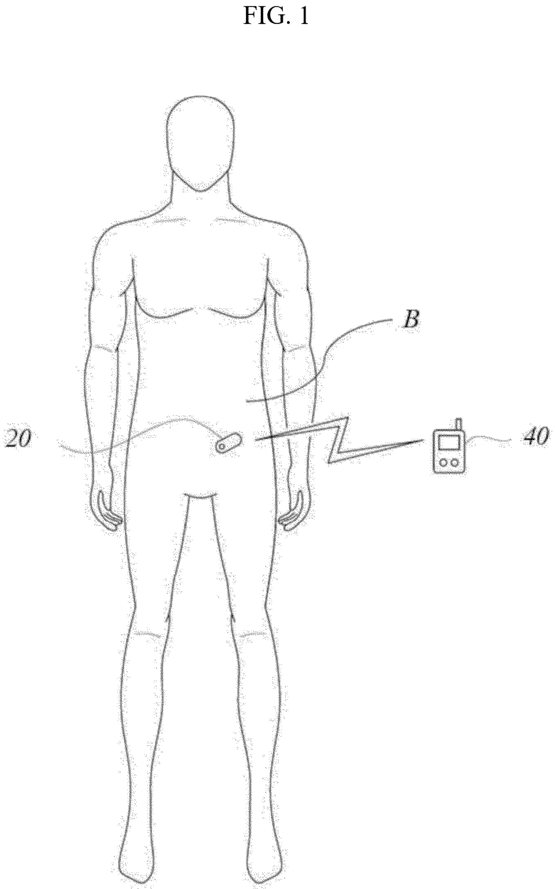

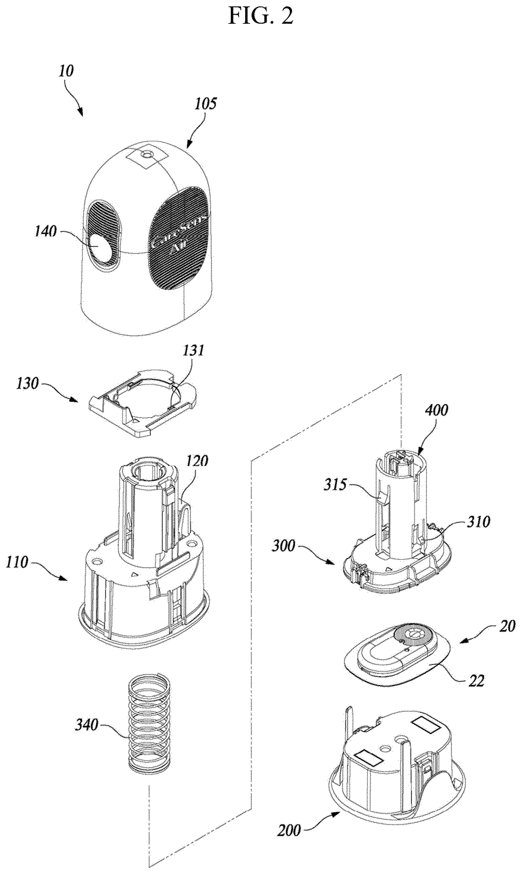

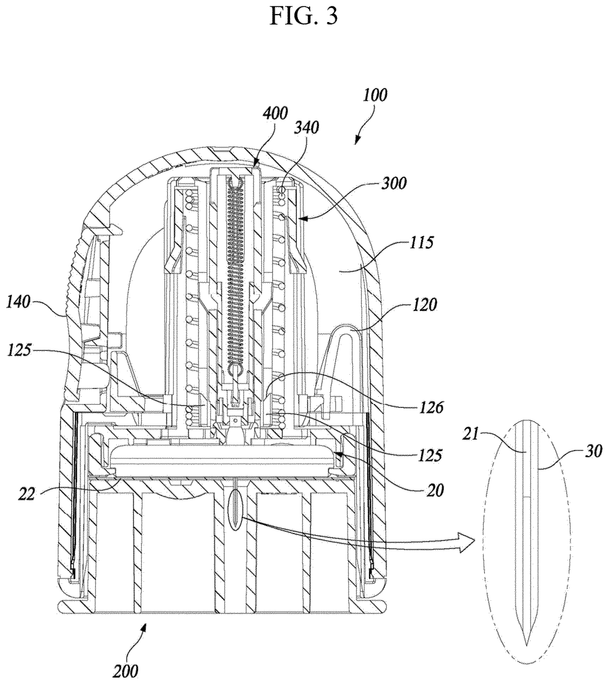

FIG. 1 is illustrating an example of usage of a blood glucose monitoring, and FIG. 2 is an exploded perspective view of an applicator for a blood glucose monitoring according to an example embodiment of the present disclosure, and FIG. 3 is a cross-sectional view illustrating a body-attachable unit installed in an applicator for a blood glucose monitoring according to an example embodiment of the present disclosure.

An applicator 10 for a blood glucose monitoring according to an example embodiment of the present disclosure is to attach a body-attachable unit 20 to measure blood glucose to a body B. A blood glucose monitoring to which the applicator 10 for the blood glucose monitoring according to an example embodiment of the present disclosure is applied may refer to a continuous glucose monitoring (CGM) system.

As shown in FIG. 1, the body-attachable unit 20 may be attached to the body B by the applicator 10 to periodically measure blood glucose from the body B and wirelessly transmit measured data to an external terminal 40. The body-attachable unit 20 may comprise a sensor member 21, a portion of which is inserted into the body, an adhesive tape 22 to attach to the body, a wireless communication chip that may wirelessly communicate with the external terminal 40, and a battery, to measure blood glucose and wirelessly transmit measured data.

The applicator 10 according to an example embodiment of the present disclosure may have a structure in which the body-attachable unit 20 may be installed. The applicator 10 may eject the body-attachable unit 20 outside by being operated through user operation. The body-attachable unit 20 may be installed within the applicator 10 and attached to the body B by being ejected from the applicator 10 according to an operation of the applicator 10.

The applicator 10 according to an example embodiment of the present disclosure may be provided to a user in a state where the body-attachable unit 20 is installed within, but the present disclosure is not limited thereto.

As the body-attachable unit 20 is provided with the adhesive tape 22 on one side, the body-attachable unit 20 may be attached to the body B of the user using the adhesive tape 22. The body-attachable unit 20 may be installed in the applicator 10 in a state in which the adhesive tape 22 is covered with a release liner. One portion of the release liner may be attached to a protective cap 200 to be removed in a process in which the protective cap 200 of the applicator 10 is separated from a main case 100.

The applicator 10 may have a structure to fix the body-attachable unit 20 in a state of being installed within and to release a holding force for the body-attachable unit 20 in a state where the body-attachable unit 20 is ejected. In the state in which the body-attachable unit 20 is installed within the applicator 10, the body-attachable unit 20 may be maintained in a fixed state to the applicator 10. And after the body-attachable unit 20 is ejected from the applicator 10, the body-attachable unit 20 may remain on the body B of the user, being separated from the applicator 10.

In a process in which the body-attachable unit 20 is ejected from the applicator 10, a needle 30 may be coupled to be separable to the body-attachable unit 20 to allow the sensor member 21 to be stably inserted into the body B of the user. The needle 30 may be configured to surround first end portion of the sensor member 21 and to be inserted together with the sensor member 21 into the body, so that the first end portion of the sensor member 21 is stably inserted into the body B.

The needle 30 may be coupled to the body-attachable unit 20 to penetrate the body-attachable unit 20 in a thickness direction. One end of the needle 30 may be in a sharp form to penetrate skin of the body B of the user and to be smoothly inserted into the body B of the user, and on another end of the needle 30 is provided with a needle head 31. The needle 30 may play a role to support the sensor member 21 to be stably inserted into the body B by penetrating the skin of the body B of the user before the sensor member 21 when the body-attachable unit 20 is ejected from the applicator 10. After the body-attachable unit 20 is attached to the body B of the user, the needle 30 may be separated from the body B of the user by a needle separation unit 400 of the applicator 10.

Hereinafter, detailed configuration of the applicator 10 according to an example embodiment of the present disclosure is described in more detail.

The applicator 10 according to an example embodiment of the present disclosure may comprise the main case 100 where a pressing button 140 is installed on one side to be pressed and operated by the user, a plunger 300 supporting the body-attachable unit 20 and movably installed within the main case 100, a plunger spring 340 applying an elastic force to the plunger 300, and the needle separation unit 400 to separate the needle 30 that is coupled to the body-attachable unit 20 from the body-attachable unit 20.

The plunger 300 may eject the body-attachable unit 20 outside by moving from a first position P1 to a second position P2 within the main case 100. Here, the first position P1 may refer to an upper side within the main case 100, and the second position P2 may refer to a lower side within the main case 100. Thus, a direction moving from the first position P1 to the second position P2 may be defined as an external ejection direction. On first end portion of the main case 100, the protective cap 200 is coupled to be separable to protect the body-attachable unit 20 within the main case 100.

The protective cap 200 is coupled to the main case 100 not to expose the body-attachable unit 20 outside in a state in which the body-attachable unit 20 is coupled to the plunger 300. The user may attach the body-attachable unit 20 to the body by operating the applicator 10 only after separating the protective cap 200.

The protective cap 200 may not only protect the body-attachable unit 20 that is inserted within the main case 100 not to be exposed outside, but also perform a support function for the body-attachable unit 20. The protective cap 200 may improve overall structural stability of the applicator 10 by being coupled to the main case 100.

The main case 100 may comprise an outer case 105, on one side of which the pressing button 140 is installed, and an inner case 110 that is formed to guide a straight path of the plunger 300 by being coupled inside the outer case 105. Inside the main case 100, an accommodating space 115 may be formed to accommodate the plunger 300, the needle separation unit 400, and the body-attachable unit 20, and the like. The accommodating space 115 may be opened to the outside, and the body-attachable unit 20 may be ejected outside through the open portion of the accommodating space 115. The opened portion of the accommodating space 115 may be opened and closed by the protective cap 200.

The pressing button 140 may be pressed to be operated by the user. A shooting plate 130, which moves in response to a pressing operation of the pressing button 140, may be installed to be movable within the main case 100.

The shooting plate 130 may move while being seated in and supported by the inner case 110. The shooting plate 130 may slide to move in response to the pressing operation of the pressing button 140. The plunger 300 may be engaged with the shooting plate 130 at the first position P1 and disengaged from the shooting plate 130 as the shooting plate 130 moves. When disengaged from the shooting plate 130, the plunger 300 may move to the second position P2 by the elastic force of the plunger spring 340.

The pressing button 140 may be modified into other various structures that may operate the shooting plate 130 according to an operation of the user, in addition to the illustrated pressing operation manner.

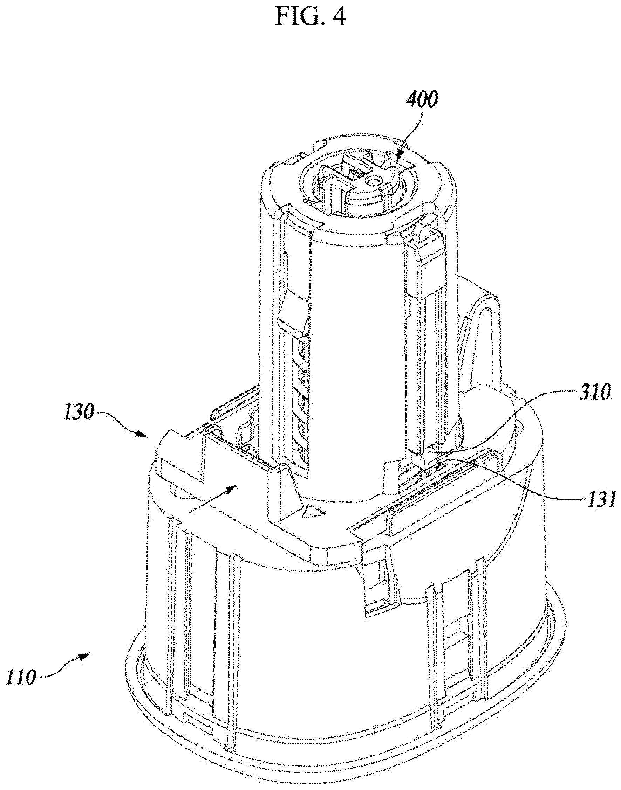

As shown in FIG. 4, the shooting plate 130 may be provided with a supporting portion 131 on one side, with which an engaging hook 310 of the plunger 300 may be engaged. The plunger 300 may be fixed at the first position P1 as the engaging hook 310 is caught on the supporting portion 131. The plunger 300 may move to the second position P2 when the engaging hook 310 is disengaged from the supporting portion 131 as the shooting plate 130 is moved by the pressing button 140.

A specific form of the shooting plate 130 or the engaging structure of the shooting plate 130 and the plunger 300 is not limited to what is illustrated but may be modified in various ways.

The inner case 110 may be provided with a return member 120 that elastically supports the shooting plate 130. The return member 120 may apply an elastic force to the shooting plate 130 in the opposite direction to a pressing direction of the pressing button 140. The shooting plate 130 may maintain a stable engaging state with the engaging hook 310 of the plunger 300 by being supported by the return member 120.

The plunger 300 is installed within the main case 100 to support the body-attachable unit 20. The plunger 300 may eject the body-attachable unit 20 from the main case 100 by moving from the first position P1 to the second position P2 by the elastic force of the plunger spring 340. The plunger 300 may be provided with a plunger projection 315. The plunger projection 315 may limit a range of movement of the plunger 300. The movement of the plunger 300 may be limited in such a manner that the plunger projection 315 is engaged with one side of the inner case 110 when the plunger 300 moves to the second position P2. Accordingly, the plunger 300 may move only to the second position P2 and stop by the elastic force of the plunger spring 340 and not slip out of the main case 100.

A sensor accommodating portion 320 into which the body-attachable unit 20 is inserted may be formed on first end portion of the plunger 300. The body-attachable unit 20 may be installed in the sensor accommodating portion 320 and may move from the first position P1 to the second position P2 together with the plunger 300. When the plunger 300 and the body-attachable unit 20 move to the second position P2, the sensor member 21 of the body-attachable unit 20 may be inserted into the body B of the user.

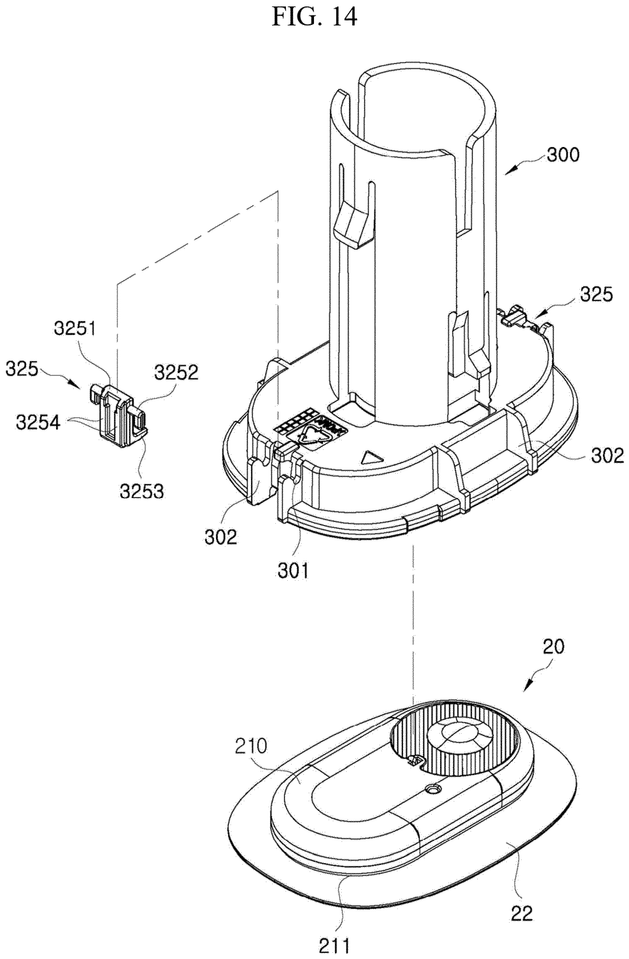

An edge of the sensor accommodating portion 320 may be provided with a fixing hook 325 that fixes the body-attachable unit 20 by being engaged with an edge of the body-attachable unit 20 that is inserted into the sensor accommodating portion 320. The fixing hook 325 may be coupled to be pivotable to the plunger 300 and may fix the body-attachable unit 20 by being engaged with an edge of the body-attachable unit 20 in a state in which the plunger 300 is positioned at the first position P1. In addition, the fixing hook 325 may be disengaged from the body-attachable unit 20 in a process in which the user separates the applicator 10 from the body-attachable unit 20 that is attached to the body B of the user, in a state where the plunger 300 is in the second position P2. A detailed description with regard to this will be described later mainly with reference to FIGS. 14 to 21.

In addition, a through hole 335 through which the needle 30 may pass may be formed in the plunger 300. The through hole 335 may be provided within the plunger 300 to connect to the sensor accommodating portion 320. The needle 30 may pass the through hole 335 and be coupled to the body-attachable unit 20 that is installed in the sensor accommodating portion 320.

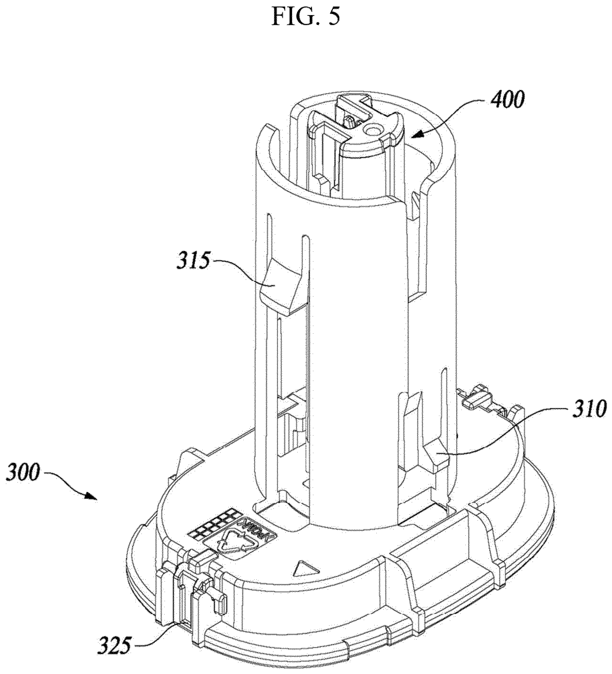

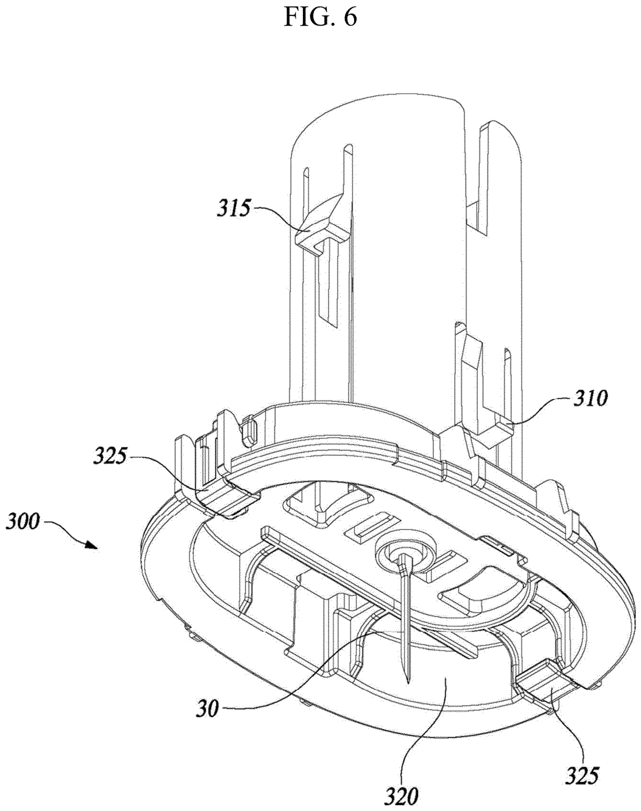

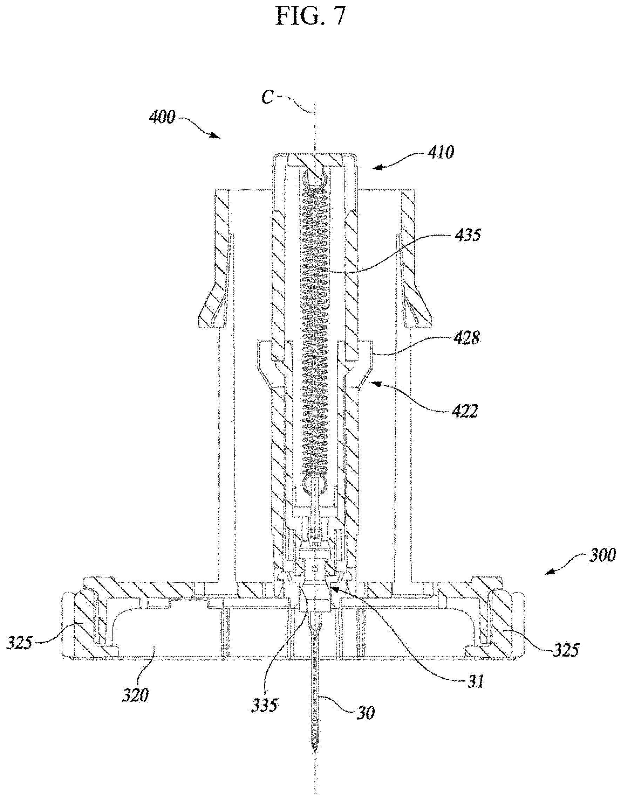

Referring to FIGS. 5 to 7, the needle separation unit 400 may separate the needle 30 that is coupled to the body-attachable unit 20 from the body B of the user as the body-attachable unit 20 moves from the first position P1 to the second position P2. The needle separation unit 400 may comprise a support column 410 that is fixed to the plunger 300, a needle holder 422 that is coupled to the needle head 31 by installed to be movable in the support column 410, and a needle separation spring 435 elastically supporting the needle holder 422.

The support column 410 may be fixed to the plunger 300 and may move from the first position P1 to the second position P2 together with the plunger 300, and the needle separation spring 435 may be installed to apply an elastic force to the needle holder 422 in a direction separating the needle holder 422 from the body-attachable unit 20, and the needle holder 422 may be fixed in a state of being engaged with the support column 410, and a trigger 428 may be formed on an outer surface to protrude outside the support column 410.

At this point, a pressing portion 125 that may make contact with the trigger 428 under pressure may be formed in the inner case 110 while the support column 410 moves from the first position P1 to the second position P2 together with the plunger 300.

Accordingly, the trigger 428 may be pressed by the pressing portion 125 in a process where the plunger 300 moves to the second position according to an operation of the applicator, and accordingly, as the needle holder 422 is disengaged from the support column 410, the needle holder 422 and the needle 30 may move upward by the elastic force of the needle separation spring 435 and be separated from the body. In this state, when the applicator is separated and removed from the body-attachable unit 20, only the body-attachable unit 20 may be left attached to the body.

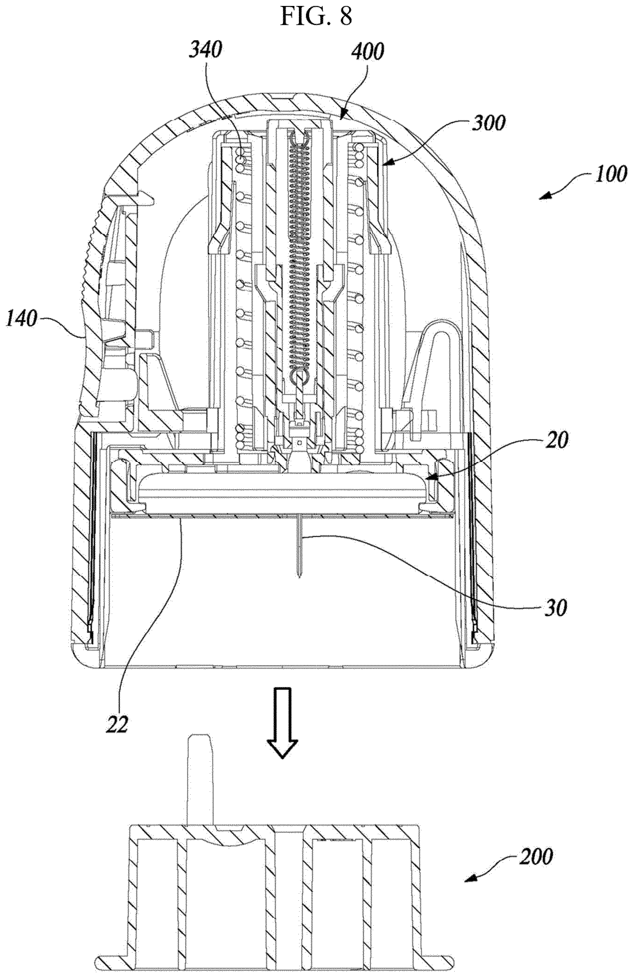

Next, a process of attaching the body-attachable unit 20 to the body B of the user by using the applicator 10 for the blood glucose monitoring according to an example embodiment of the present disclosure will be described with reference to FIGS. 8 to 13.

First, the protective cap 200 may be separated as illustrated in FIG. 8. In the process of separating the protective cap 200, the release liner of the body-attachable unit 20 may be separated together with the protective cap 200 to expose the adhesive tape 22 of the body-attachable unit 20.

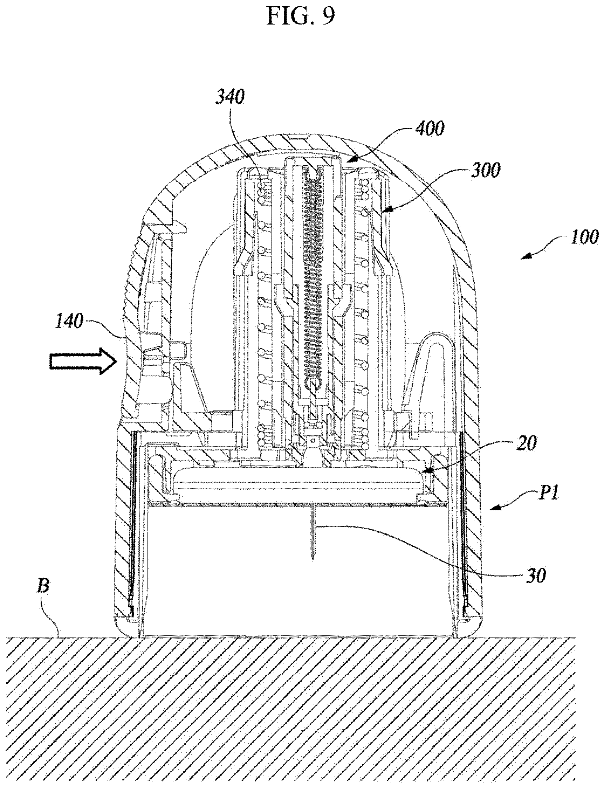

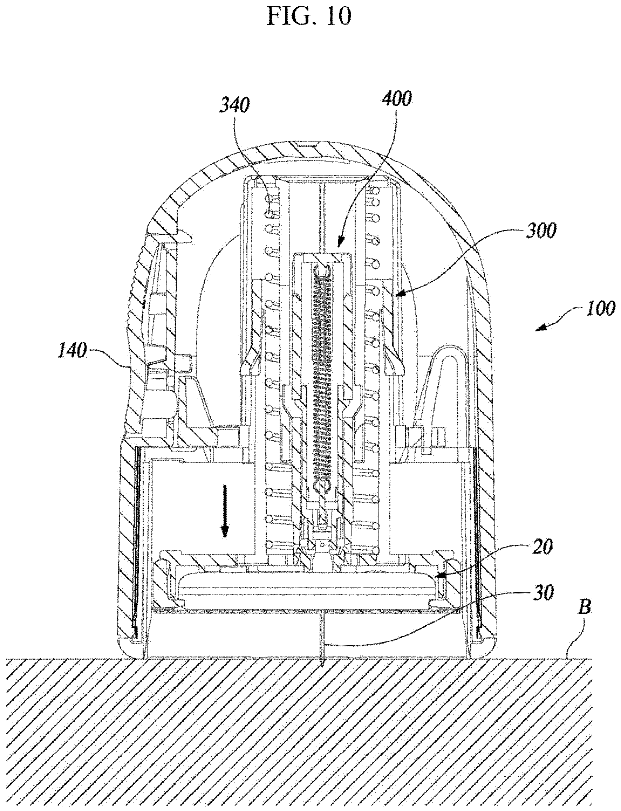

Subsequently, as illustrated in FIG. 9, the applicator 10 may be positioned on the body B of the user to whom the body-attachable unit 20 is attached, and the pressing button 140 may be pressed and operated. When the pressing button 140 is operated, the shooting plate 130 may move, and a holding force for the plunger 300 that is fixed at the first position P1 may be released. When the holding force for the plunger 300 is released by the shooting plate 130, as shown in FIG. 10, the plunger 300 that is provided with the body-attachable unit 20 may move to the second position P2 by the plunger spring 340, and the needle 30 and the sensor member 21 of the body-attachable unit 20 may penetrate the skin of the user and be inserted into the body B.

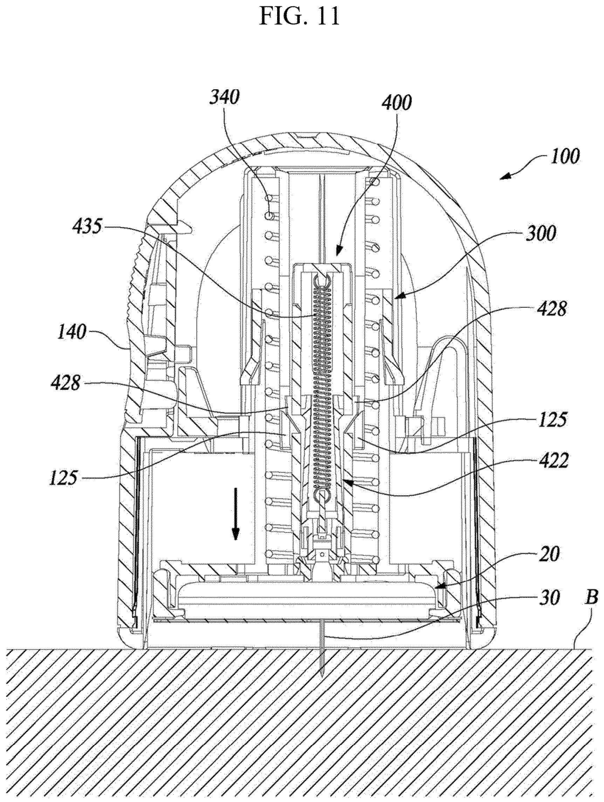

As illustrated in FIG. 11, when the trigger 428 of the needle separation unit 400 reaches the pressing portion 125 of the main case 100 while the plunger 300 moves to the second position P2, the trigger 428 may be pressed by the pressing portion 125 and may release a holding force for the needle holder 422. At the same time, the needle holder 422 may move in a direction away from the body B of the user by the elastic force of the needle separation spring 435.

As the needle holder 422 moves by the elastic force of the needle separation spring 435, the needle 30 that is inserted into the body B together with the sensor member 21 of the body-attachable unit 20 may be separated from the body-attachable unit 20 as being pulled by the needle holder 422.

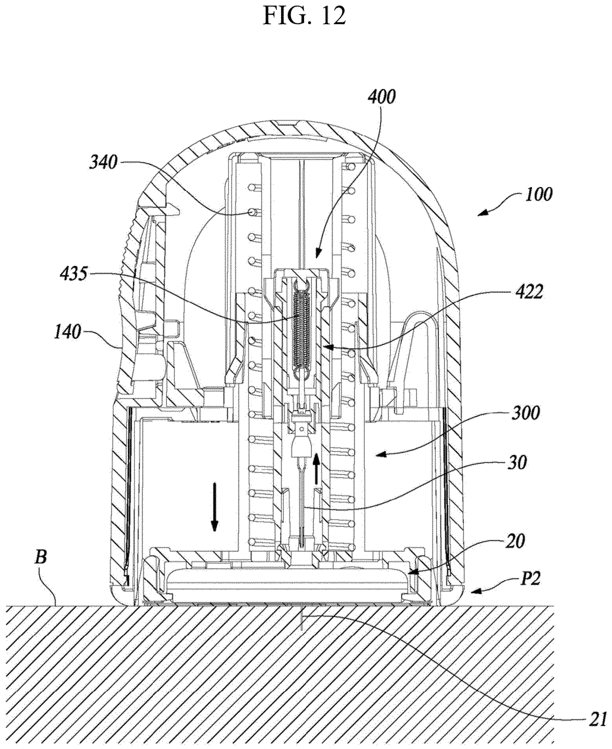

Continuously, as illustrated in FIG. 12, the plunger 300 may move to the second position P2 by the elastic force of the plunger spring 340 and stop. At this point, the body-attachable unit 20 may be attached to the body B of the user by the adhesive tape 22.

A point when the needle holder 422 separates the needle 30 from the body-attachable unit 20 while the body-attachable unit 20 moves to the second position P2 may be set to an appropriate point after guiding the needle 30 to penetrate the skin of the body B of the user and insert the sensor member 21 into the body B of the user. To this end, the pressing portion 125 may be disposed at an appropriate position in a moving path of the needle holder 422 depending on a length of the needle 30 and the like.

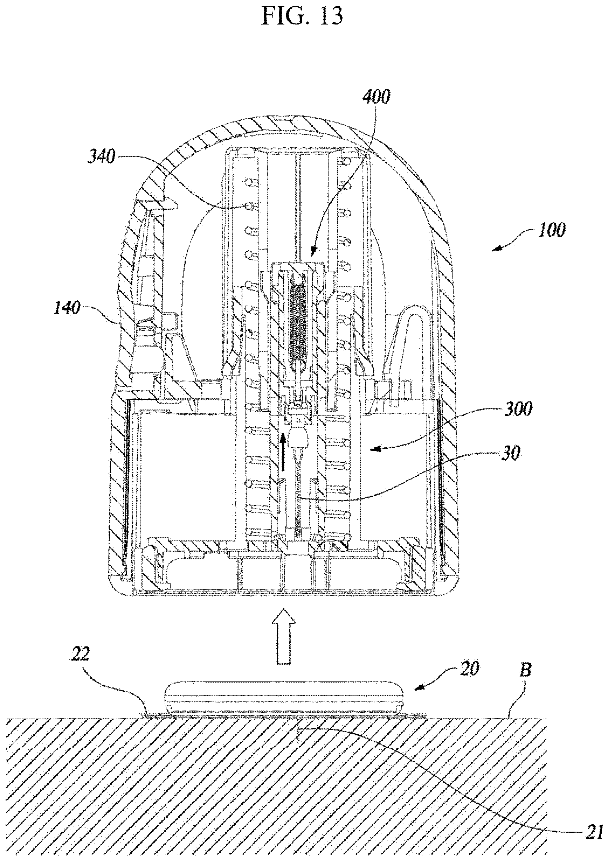

As illustrated in FIG. 13, after the body-attachable unit 20 is attached to the body B of the user, the attachment work for the body-attachable unit 20 may be completed when the applicator 10 is separated from the body-attachable unit 20.

Subsequently, the body-attachable unit 20 may measure blood glucose of the user and transmit measured information to the external terminal 40.

Next, a coupling structure of the plunger 300 and the body-attachable unit 20 is described in more detail mainly with reference to FIGS. 14 to 21.

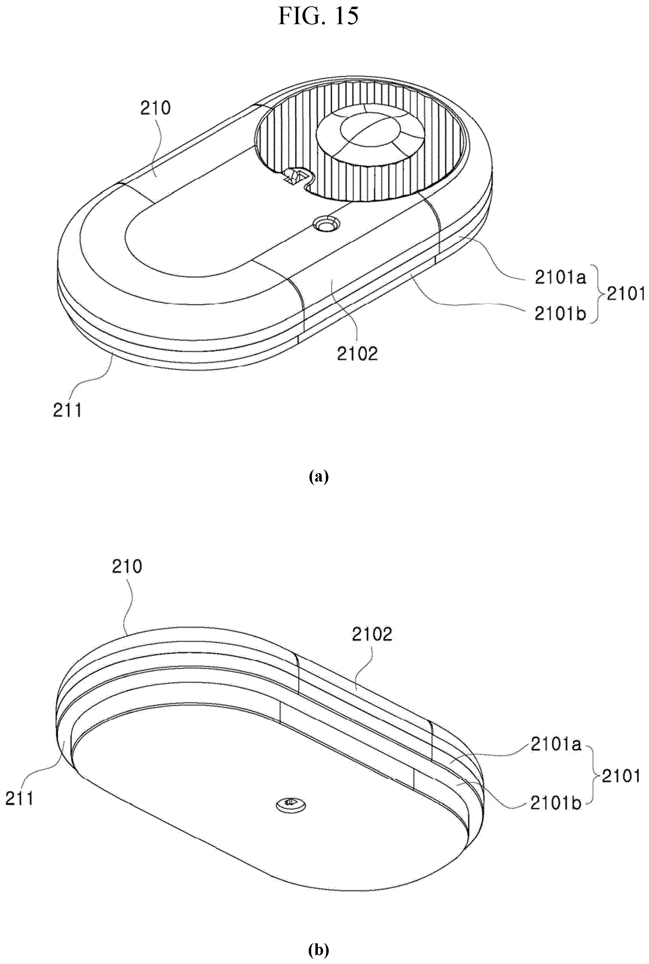

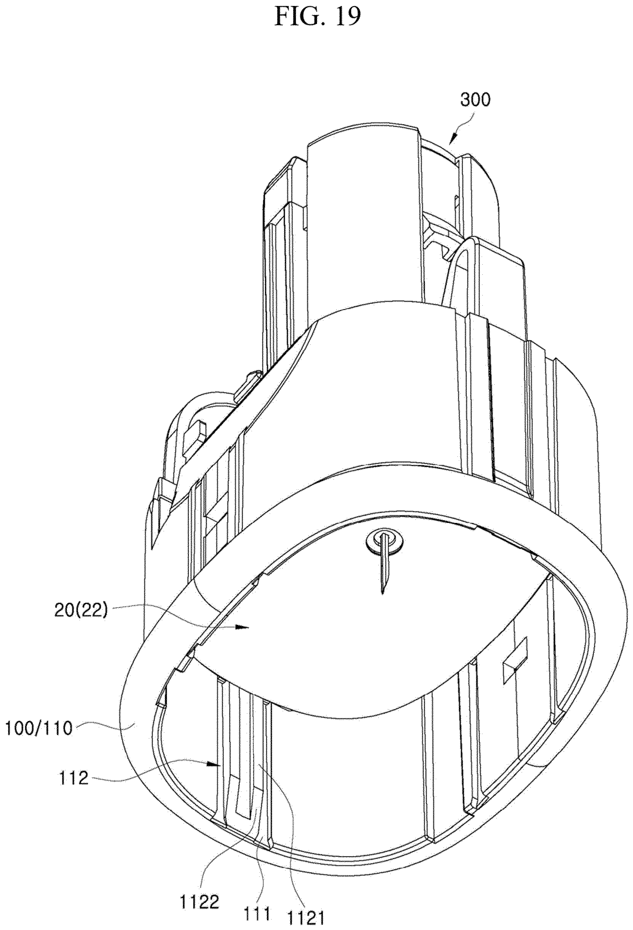

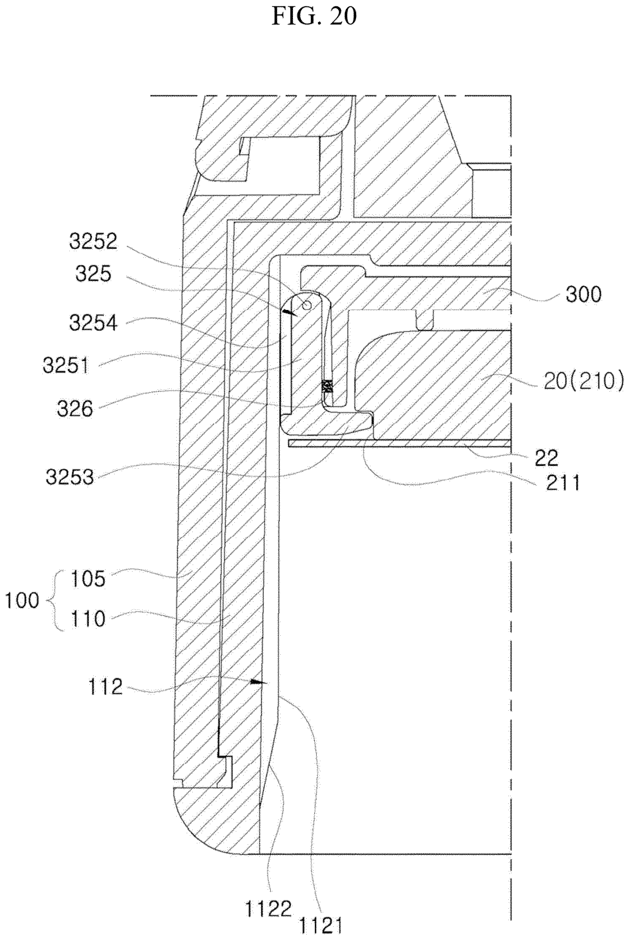

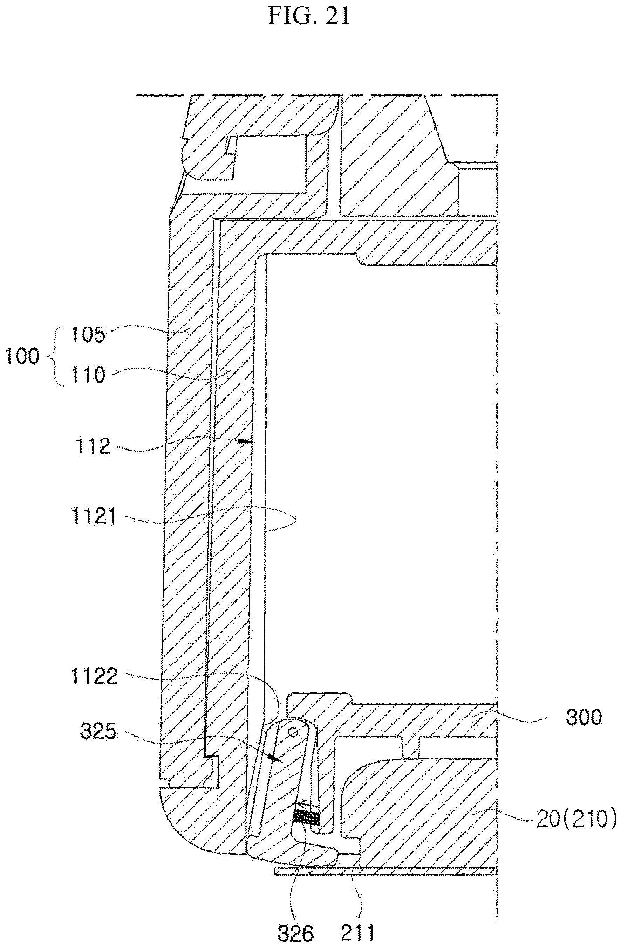

FIG. 14 is a perspective view illustrating a coupling structure of a plunger and a body-attachable unit according to an example embodiment of the present disclosure, and FIGS. 15 through 18 are exemplary diagrams illustrating various forms of a recess portion formed on a body-attachable unit according to an example embodiment of the present disclosure, and FIG. 19 is a schematic perspective view illustrating a form of a hook guide portion formed on an inner surface of a main case according to an example embodiment of the present disclosure, and FIG. 20 is a cross-sectional view illustrating a coupling structure of a plunger and a body-attachable unit at a first position within an applicator according to an example embodiment of the present disclosure, and FIG. 21 is a cross-sectional view illustrating a coupling structure of a plunger and a body-attachable unit at a second position within an applicator according to an example embodiment of the present disclosure.

As described above, the plunger 300 may move from the first position P1 to the second position P2 within the applicator 10, and the body-attachable unit 20, inserted and accommodated in the sensor accommodating portion 320 of the plunger 300, may move from the first position P1 to the second position P2 together with the plunger 300.

At this point, the plunger 300 may be coupled to the fixing hook 325 that is coupled to the body-attachable unit 20 at the first position P1 and decoupled from the body-attachable unit 20 at the second position P2, and a recess portion 211 that may be engaged with and coupled to the fixing hook 325 may be formed on the body-attachable unit 20.

In such a manner, the fixing hook 325 may be installed in the plunger 300 to couple and decouple the body-attachable unit 20, but as needed, the body-attachable unit 20 may be simply inserted and coupled to the plunger 300 without the fixing hook 325, and the coupling structure of the body-attachable unit 20 and the plunger 300 may be modified in various ways. Hereinafter, a structure in which the fixing hook 325 is installed in the plunger 300 will be mainly described for convenience of description.

The body-attachable unit 20 may comprise a housing 210 constituting an outer form and the sensor member 21 (see FIG. 3), one end of which protrudes downward outside the housing 210, and another end of which is installed within the housing 210, and the recess portion 211 is formed on an outer surface of the housing 210. More specifically, the housing 210 may include an upper frame 2102 and a lower frame 2101 that is disposed under the upper frame 2102 and having a surface attached to the body, and the recess portion 211 may be formed on an outer surface of the lower frame 2101.

The fixing hook 325 may be formed on an edge of the sensor accommodating portion 320 of the plunger 300 to be coupled to and fix the body-attachable unit 20 by being engaged with and coupled to the recess portion 211 of the body-attachable unit 20 that is inserted into the sensor accommodating portion 320. The fixing hook 325 may be coupled to be pivotable around a hinge axis 3252, and in a state in which the plunger 300 is positioned at the first position P1, the fixing hook 325 may be engaged with and coupled to the body-attachable unit 20 by being pressed in an inward direction by the inner case 110, and in a state in which the plunger 300 is positioned at the second position P2, the fixing hook 325 may be released from pressure and disengaged and decoupled from the body-attachable unit 20. In such a manner, to press and release pressure for the fixing hook 325, a hook guide portion 112 may be formed in the main case 100, more specifically, the inner case 110 of the main case 100.

The fixing hook 325 may comprise, as illustrated in FIG. 14, a pivoting body 3251, on an upper portion of which the hinge axis 3252 is formed in a horizontal direction and a hook projection 3253 formed on a lower portion of the pivoting body 3251 to protrude to be engaged with the recess portion 211 of the body-attachable unit 20. On one surface (an opposite surface of the direction where the hook projection 3253 protrudes) of the pivoting body 3251, a pressing guide 3254 may be formed to protrude to allow a more stable pressure state by the hook guide portion 112 of the inner case 110. The pressing guide 3254 may be formed to be long in an up and down direction on one surface of the pivoting body 3251.

A hinge axis coupling groove 301 may be formed on the plunger 300 to allow the hinge axis 3252 of the fixing hook 325 to be coupled to be pivotable, and the hinge axis coupling groove 301 may be formed in a concavely recessed shape on a top of a moving guide protruding portion 302 that is formed on an outer surface of the plunger 300. The moving guide protruding portion 302 may be formed on the outer surface of the plunger 300 to guide a moving path of the plunger 300 in a process in which the plunger 300 moves from the first position P1 to the second position P2, and in response to this, a guide groove 111 may be formed on an inner surface of the inner case 110 to guide the moving guide protruding portion 302 of the plunger 300 to be inserted.

A plurality of fixing hooks 325 may be coupled to the plunger 300, and in an example embodiment of the present disclosure, as illustrated in FIG. 14, two fixing hooks 325 may be installed to face each other on both lateral end portions of the plunger 300. Of course, the form may be modified in various ways, for example, with four fixing hooks 325 provided in a form where each pair faces each other.

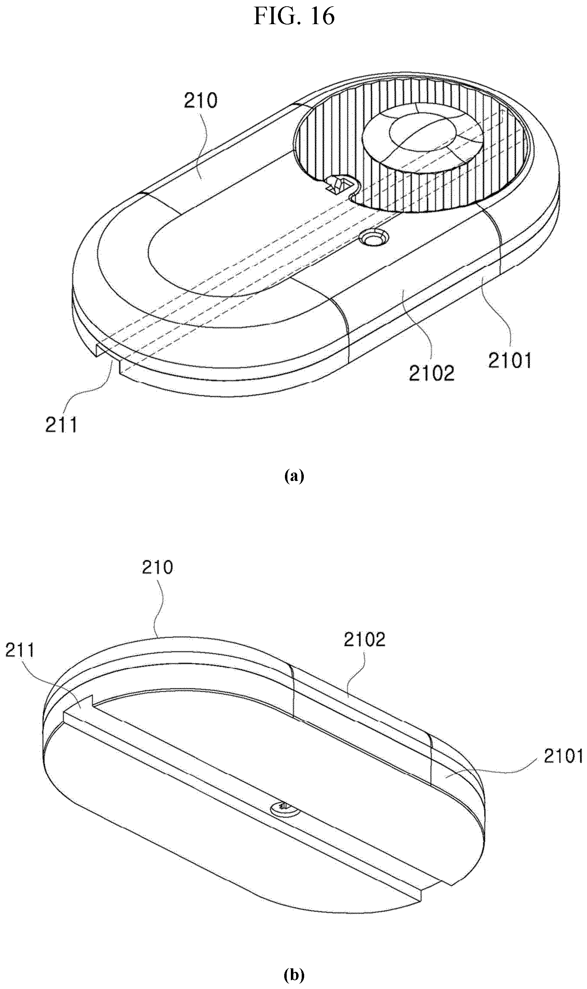

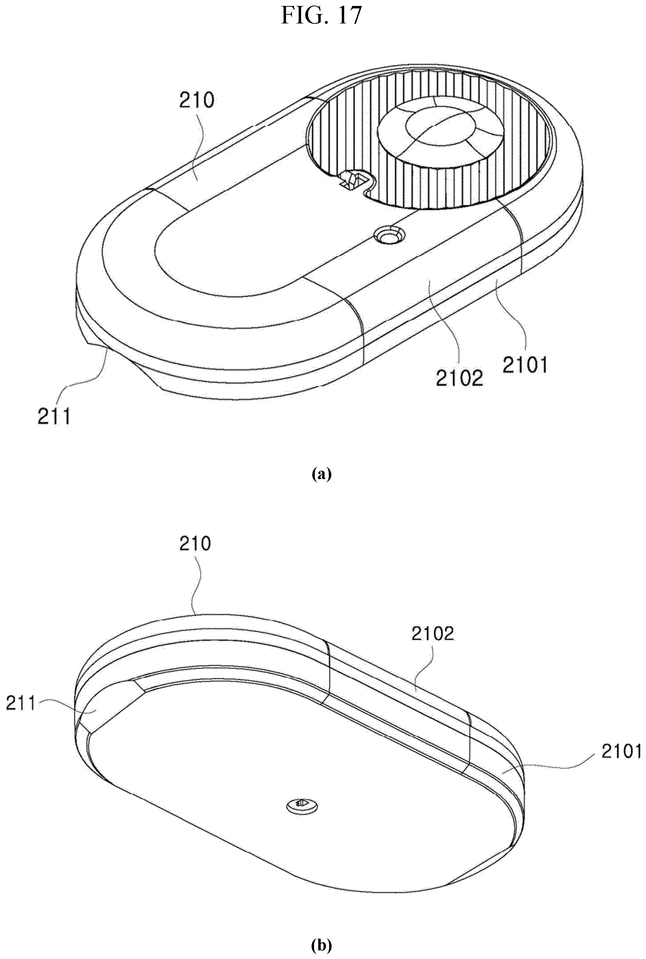

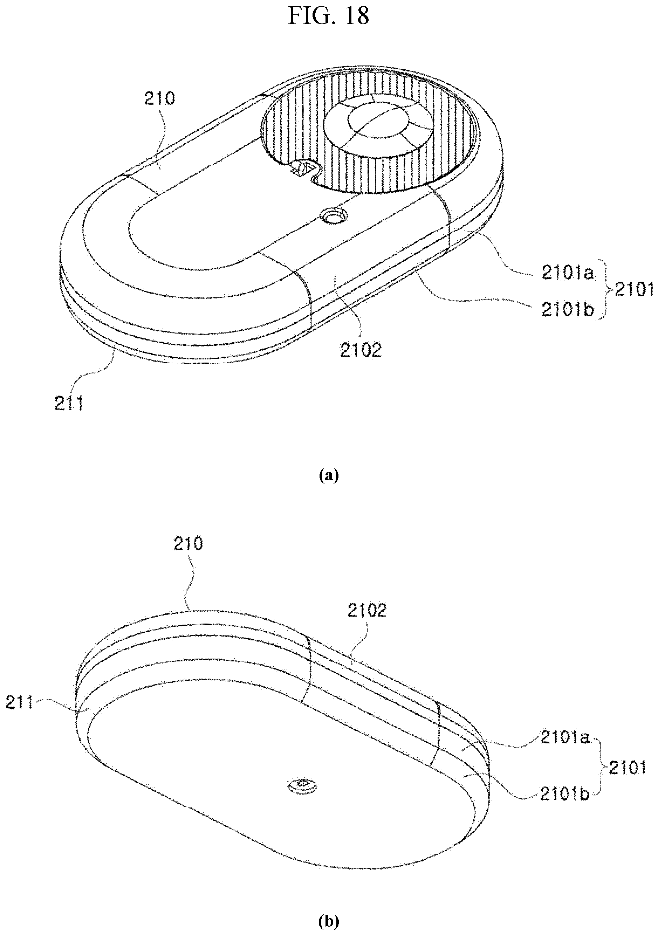

The recess portion 211 that is formed on the body-attachable unit 20 may be formed on the outer surface of the housing 210, and as illustrated in FIGS. 15 and 16, formed in a concave form with a step on the outer surface of the housing 210, and as illustrated in FIGS. 17 and 18, formed in a shape having an inclined surface as a lower corner portion on the outer surface of the housing 210 is chamfered. Of course, the form may be modified in various ways to allow the fixing hook 325 to be engaged.

First, the recess portion 211 illustrated in FIG. 15 may be consecutively formed throughout the entire section along a perimeter of a lower portion of the outer surface of the housing 210, and the outer surface of the housing 210 may be formed in a concave shape with a step. More specifically, as aforementioned, the housing 210 may include the upper frame 2102 and the lower frame 2101, and the lower frame 2101 may comprise a first area 2101a at an upper portion and a second area 2101b at a lower portion. The first area 2101a may be coupled to a side wall of the upper frame 2102 to be connected in a planar manner without a step, and the second area 2101b may be positioned at a lower portion of the first area 2101a and formed with a step in an inward direction from the first area 2101a.

In such a manner, as the recess portion 211 is formed in a concave shape with a step, the fixing hook 325 may be easily engaged with the step portion of the recess portion 211. In addition, as a plurality of fixing hooks 325 are coupled, a plurality of recess portions 211 may be formed in response, but in FIG. 15, as the recess portion 211 is consecutively formed in an integrated form on the outer surface of the housing 210, the plurality of fixing hooks 325 may be simultaneously engaged with and coupled to one recess portion 211. Accordingly, it is not required to manufacture a plurality of recess portions 211, and particularly, even when the number or a position of the fixing hook 325 changes, a position of the recess portion 211 of the body-attachable unit 20 does not need to be changed, and an identical product may be used to easily manufacture and manage a component.

Meanwhile, the recess portion 211 may be, as illustrated in FIG. 16, consecutively formed from one side of the outer surface of the housing 210 through a bottom surface to another side of the outer surface, and the outer surface of the housing 210 may be formed in a concave form with a step. As described in FIG. 15, the housing 210 may include the upper frame 2102 and the lower frame 2101, and at this point, the recess portion 211 may be consecutively formed to be extended from one side of the outer surface of the lower frame 2101 through the bottom surface to another side of the outer surface as illustrated in FIG. 16. The recess portion 211 may be formed to cross the bottom surface of the housing 210 in both end directions in a concave groove form concavely recessed on the bottom surface.

The recess portion 211 may also be in an integrated form to be connected to each other, and a plurality of fixing hooks 325 may be simultaneously engaged with one recess portion 211. In an example illustrated in FIG. 16, two fixing hooks 325 facing each other may be engaged with and coupled to both lateral end portions of the integrated recess portion 211.

The recess portion 211 illustrated in FIG. 17 may be formed in a form having an inclined surface as a lower corner portion of the outer surface of the lower frame 2101 of the housing 210 is chamfered and formed at a position corresponding to a position of the fixing hook 325. For example, according to an example embodiment of the present disclosure, two recess portions 211 facing each other may be formed to correspond to a position of the two fixing hooks 325 facing each other. In such a manner, as the recess portion 211 is formed in a manner in which a lower corner portion of the outer surface of the housing 210 is chamfered, manufacture may be easy because a separate molding process is not required, and an engaging function with the fixing hook 325 may also be stably performed.

The recess portion 211 in a chamfered shape may be, as illustrated in FIG. 18, consecutively formed along an entire perimeter of a lower corner on the outer surface of the housing 210. More specifically, the housing 210 may include the upper frame 2102 and the lower frame 2101, and the lower frame 2101 may comprise the first area 2101a at an upper portion and the second area 2101b at a lower portion as aforementioned, and the recess portion 211 may be formed in a form having a inclined surface as an outer lower corner formed at the second area 2101b of the lower frame 2101 is chamfered along an entire perimeter.

In this case, as with the recess portion 211 illustrated in FIG. 15, a plurality of fixing hooks 325 may be simultaneously engaged with one recess portion 211, and even when the number or a position of the fixing hook 325 changes, a position of the recess portion 211 of the body-attachable unit 20 does not need to be changed, and an identical product may be used to easily manufacture and manage a component.

Accordingly, as illustrated in FIGS. 15, 16, and 18, the recess portion 211 according to an example embodiment of the present disclosure may be formed to be extended in a consecutive form along a position at which each of a plurality of fixing hooks 325 are engaged, allowing the plurality of fixing hooks 325 are simultaneously engaged, and through this, it is possible to easily manufacture and manage a component.

In FIG. 19, a form of the hook guide portion 112 formed on an inner surface of the inner case 110 of the main case 100 is illustrated. The hook guide portion 112 may be formed to press the fixing hook 325 to be engaged with the recess portion 211 of the body-attachable unit 20 or to release pressure, depending on a position of the plunger 300.

The hook guide portion 112 may be formed to protrude in an inner central direction on an inner surface of the inner case 110 and formed to be long in an up and down direction. The hook guide portion 112 may be formed to comprise a protruding surface 1121 and a concave surface 1122 on an inner surface, and the concave surface 1122 may be formed to be extended on a lower end of the protruding surface 1121 and is inclined in a shape in which a protruding height decreases towards the lower end. On both sides of the hook guide portion 112, the guide groove 111 may be formed to allow the moving guide protruding part 302 of the plunger 300 to be guided and inserted.

As illustrated in FIGS. 20 and 21, the protruding surface 1121 of the hook guide portion 112 may be formed to press the fixing hook 325, and the concave surface 1122 to release pressure for the fixing hook 325. The protruding surface 1121 may be formed to press the fixing hook 325 while the plunger 300 moves from the first position P1 to the second position P2, and more specifically, to press the pressing guide 3254 in contact with the pressing guide 3254 of the fixing hook 325. The concave surface 1122 may be formed to release pressure for the fixing hook 325 in a state in which the fixing hook 325 is in the second position P2 together with the plunger 300. In other words, as the protruding surface 1121 protrudes relatively in an inward direction, the fixing hook 325 may be pressed toward the recess portion 211 in a process of the plunger 300 moving from the first position P1 to a section right before moving to the second position P2, and as the concave surface 1122 is relatively in a concave form, the fixing hook 325 may be released from pressure in a state in which the plunger 300 is positioned at the second position P2. At this point, the fixing hook 325 may be engaged with or disengaged from the recess portion 211 of the body-attachable unit 20 as pivoting around the hinge axis 3252 depending on a pressed state.

According to the structure, the fixing hook 325 may be pressed in an inward direction by the protruding surface 1121 of the hook guide portion 112 while the plunger 300 moves from the first position P1 to the second position P2 as illustrated in FIG. 20, and the fixing hook 325 may be engaged with the recess portion 211 of the body-attachable unit 20 that is inserted and accommodated in the sensor accommodating portion 301 of the plunger 300 as pivoting around the hinge axis 3252. When the plunger 300 moves in a straight line to the second position P2 as illustrated in FIG. 21, the fixing hook 325 may be positioned at a position corresponding to the concave surface 1122 of the hook guide portion 112. Accordingly, as the fixing hook 325 may be released from pressure by the concave surface 1122 of the hook guide portion 112, the fixing hook 325 may be pivotable around the hinge axis 3252 and be disengaged from the body-attachable unit 20. In this state, in other words, in a state in which the plunger 300 is in the second position P2 together with the body-attachable unit 20, the body-attachable unit 20 may be attached to the body by the adhesive tape 22 (see FIG. 12), and when the applicator 10 is separated and removed in a direction separating from the body (see FIG. 13), as the fixing hook 325 and the body-attachable unit 20 are disengaged from each other, only the applicator 10 may be separated and removed from the body, and only the body-attachable unit 20 may be attached to and remained on the body.

Meanwhile, in a state where the plunger 300 is in the second position P2, as the fixing hook 325 may be positioned at a position corresponding to the concave surface 1122 of the hook guide portion 112, technically, the fixing hook 325 may only be released from pressure by the protruding surface 1121 and may not forcibly pivot in a disengaging direction.

In this case, as the fixing hook 325 may freely pivot around the hinge axis 3252, an engaging state between fixing hook 325 and the recess portion 211 may not be maintained but released in a process of separating and removing the applicator 10 from the body, in other words, in a process of the plunger 300 and the fixing hook 325 positioned within the applicator 10 separating from the body-attachable unit 20.

If the free pivoting state of the fixing hook 325 is not properly operated with an unknown reason, the engaging state between the fixing hook 325 and the recess portion 211 may not be smoothly released in a process of separating the applicator 10 from the body. In this case, as some of the strength separating the applicator 10 from the body may be transferred to the body-attachable unit 20, the attachment state of the body-attachable unit 20 to the body may become unstable, for example, with a portion of the adhesive tape 22 of the body-attachable unit 20 detached from the body.

In an example embodiment of the present disclosure, an elastic member 326 that applies an elastic force to the fixing hook 325 in a direction where the fixing hook 325 is disengaged from the recess portion 211 may be separately provided as illustrated in FIGS. 20 and 21. The elastic member 326 may be formed of an elastic rubber material disposed between the fixing hook 325 and a wall of the plunger 300, but may alternatively be formed in a coil spring form or a torsion spring form coupled to the hinge axis 3252.

As the fixing hook 325 may be elastically supported by the elastic member 326 in a direction disengaged from the recess portion 211, the fixing hook 325 may be forcibly disengaged from the recess portion 211 by forcibly pivoting due to the elastic force of the elastic member 326 when the plunger 300 moves to the second position P2. Accordingly, the state of the fixing hook 325 disengaged from the recess portion 211 may become more stable.

Meanwhile, a cross-sectional view of a form of the body-attachable unit 20 illustrated in FIG. 15 is illustrated in FIGS. 20 and 21, and although described based on this, the form of the body-attachable unit 20 illustrated in FIGS. 16 to 18 operates in the same manner, so detailed description with regard to this is omitted.

The above description is merely to describe an example of the technical idea of the present disclosure, and those skilled in the art to which the present disclosure belong may allow various changes and modifications within a range not deviating from the essential characteristics of the present disclosure. Thus, example embodiments disclosed in the present disclosure is to describe the technical idea of the present disclosure, not to limit the technical idea, and the range of the technical idea of the present disclosure is not limited to those example embodiments. The scope of the present disclosure should be construed with the following claims, and all technical ideas within a range equivalent thereto should be construed as being included in the scope of the present disclosure.

## Claims

1. A sensor applicator assembly for a blood glucose monitoring, comprising:
an applicator; and
a body-attachable unit disposed inside the applicator and comprising a housing including an upper frame and a lower frame,
wherein the lower frame comprises a recess portion formed throughout an
entire section along a perimeter direction.

2. The sensor applicator assembly for the blood glucose monitoring of claim 1,
further comprising a plunger disposed inside a main case of the applicator, wherein the plunger is coupled to a fixing hook that is coupled to the body-attachable unit at a first position inside the main case and decoupled from the body-attachable unit at a second position positioned in a downward direction from the first position.

3. The sensor applicator assembly for the blood glucose monitoring of claim 1,
wherein the body-attachable unit
comprises a sensor member, first end portion of which is disposed inside the housing including the upper frame and the lower frame, and another end portion of which protrudes outside the housing.

4. The sensor applicator assembly for the blood glucose monitoring of claim 2,
wherein first end portion of the fixing hook is coupled to the plunger and another end portion is engaged with the recess portion, and
wherein the fixing hook comprises at least one of the first end portion coupled to the plunger.

5. The sensor applicator assembly for the blood glucose monitoring of claim 1,
wherein the lower frame comprises a first area coupled to a side wall of the upper frame to be connected in a planar manner without a step and a second area formed with a step in an inward direction from the first area.

6. The sensor applicator assembly for the blood glucose monitoring of claim 1,
wherein the lower frame comprises a first area coupled to a side wall of the upper frame to be connected in a planar manner without a step and a second area formed in a shape having an inclined surface as a corner portion extended to a downward direction from the first area is chamfered.

7. The sensor applicator assembly for the blood glucose monitoring of claim 2,
wherein the plunger
comprises a sensor accommodating portion in which the body-attachable unit is inserted and accommodated, and
wherein the fixing hook
is coupled to an edge of the sensor accommodating portion and is formed to allow first end portion to be pivotable around a hinge axis to be engaged with or disengaged from the recess portion.

8. The sensor applicator assembly for the blood glucose monitoring of claim 2,
wherein the plunger moves from the first position to the second position within the main case, and
wherein a hook guide portion is formed on an inner surface of the main case to press the fixing hook in a direction in which the recess portion is engaged or to release pressure depending on a position of the plunger.

9. The sensor applicator assembly for the blood glucose monitoring of claim 1,
wherein the plunger is coupled to an elastic member that applies an elastic force to the fixing hook in a direction in which the fixing hook is disengaged from the recess portion.

10. A sensor applicator assembly for a blood glucose monitoring, comprising:
an applicator; and
a body-attachable unit disposed inside the applicator and comprising a housing including an upper frame and a lower frame,
wherein the lower frame comprises a recess portion formed in a shape extending from one side of an outer surface of the lower frame through a
bottom surface to another side of the outer surface.

11. The sensor applicator assembly for the blood glucose monitoring of claim 10,
wherein the body-attachable unit
comprises a sensor member, first end portion of which is disposed inside the housing including the upper frame and the lower frame, and another end portion of which protrudes outside the housing.

12. The sensor applicator assembly for the blood glucose monitoring of claim 10,
wherein the recess portion is formed in a concave groove shape concavely recessed on the bottom surface of the lower frame to cross a bottom surface of the housing in a direction towards both side ends.

13. The sensor applicator assembly for the blood glucose monitoring of claim 10,
further comprising a plunger disposed inside a main case of the applicator, wherein the plunger further comprises:
a fixing hook coupled to be engaged with or disengaged from the recess portion; and
an elastic member that applies an elastic force to the fixing hook in a direction in which the fixing hook is disengaged from the recess portion.
